# EUROPEAN PATENT SPECIFICATION

(11) **EP 0 440 699 B1**
(45) Date of publication and mention of the grant of the patent: **21.12.1994**
(21) Application number: 89911908.5
(22) Date of filing: 25.10.1989
(51) Int. Cl.: C12Q 1/24, C12Q 1/66, G01N 31/22

(54) **SAMPLING DEVICE**
MUSTERNAHMEANORDNUNG
DISPOSITIF DE PRELEVEMENT D'ECHANTILLONS

(30) Priority: 26.10.1988 SE 8803834
(43) Date of publication of application: 14.08.1991
(73) Proprietor: FUNKE, Hans, S-633 57 Eskilstuna (SE); HOLMBERG, Olof, S-136 71 Handen (SE); LUNDIN, Arne, S-130 54 Dalarö (SE)
(72) Inventor: FUNKE, Hans, S-633 57 Eskilstuna (SE); HOLMBERG, Olof, S-136 71 Handen (SE); LUNDIN, Arne, S-130 54 Dalarö (SE)
(74) Representative: Burman, Tore
(86) International application number: SE8900594
(87) International publication number: WO9004647

(56) References cited:
- EP-A- 128 422
- WO-A-86/07094
- US-A- 4 136 680

## Description

The present invention relates to a device for sampling and transportation of liquid media, for example milk for test on mastitis. The invention also relates to a process for sampling while maintaining the quality of the sample during transportation.

The invention refers to sampling devices by means of which liquid media can be tested with regard to for example content of bacteria, such as in regard to quantity and type of bacteria. The present invention will to a large extent be illustrated in relation to milk samples for investigating the samples with regard to contents of cells and type of bacteria, but the invention shall in no way be considered to be limited to only such application.

The conventional method of sending milk samples to a laboratory is carried out in such a manner that a few milliliters are milked into a test tube in such an aseptic way as possible, said test tube being then passed on to a laboratory. The milk sample is investigated there with regard to cell content and presence of bacteria. Present methods to determine cell content are either time and staff consuming as far as mass investigations are concerned, require expensive technical equipment or show deficiencies with regard to sensitivity and accuracy. The difficulty in milk sampling results in a large number of samples being contaminated in the sampling procedure. In view of the fact that the milk is a very efficient substrate for the bacteria, contaminating bacteria may grow during transportation and cause problems when evaluating the bacteriological test. About 25% of the samples from subclinical tests on mastitis investigated at relevant laboratories contain contaminating bacteria. Thus, if the samples are subject to transportation without efficient cooling, which in practice is almost impossible to do, these bacteria are subject to growth and result in an inaccurate bacteriological diagnosis.

A sampling device constituting an illustrative example from the state of the art, is disclosed in US-A-4 136 680. Said device defined as a specimen collecting apparatus involves a self-contained in-line transport and culture container with associated means for taking a specimen from its environment and for protecting same during transportation to a laboratory for analysis. However, in the absence of specific means for bringing or maintaining the liquid sample in a substantially dry condition this prior art apparatus is unable to prevent undesirable growth of microorganisms, such as bacteria, in the collected specimen.

The present invention has for an object to provide a device for sampling from a liquid medium, for example milk, by which the disadvantages of the conventional techniques are eliminated or at any rate substantially reduced.

For these and other objects the invention provides for such a device for sampling from a liquid medium, said device comprising a container which by a transverse partition is divided into two compartments containing at least one sampling element comprising an operational part placed in one compartment and a sampling part placed in the other compartment. The device according to the invention is characterized in that the end wall of the container in connection with said other compartment is provided with an opening opposite to the sampling member through which said member by means of the respective operational part can be pushed out of the container for exposing the sampling part and for the uptake of sample thereon and then be retracted into the container for protection during transportation.

In a preferred embodiment of the device according to the invention it is provided with an outwardly pivotal end-wall which during transportation with the sampling elements in a retracted position covers said openings so that the sampling member will be protected during transportation to a laboratory for evaluation.

Each sampling part is suitably provided with one or several detachable sampling elements of an absorbent porous material, for example of the filter paper type. It is preferred that each sampling part is provided with two such detachable sampling elements, one for bacterial analysis and another for analysis with regard to adenosin triphosphate (ATP).

For the purpose of maintaining said samples as dry as possible during transportation to the laboratory it is particularly preferred to arrange for a drying agent in said other compartment containing the sampling parts of the sampling member, said compartment being substantially closed against the environment. In such embodiment the device may suitably be provided with an openable lid designed as a box containing the drying agent and which in a closed position seals said other compartment.

The device according to the invention may suitably be provided with a cover plate which is provided with grooves and recesses placed opposite to the respective operational parts. The operational part is at its free end provided with a grip extending up through the recess for extension or retraction of the corresponding sampling part in connection with sampling. For test with regard to other inflammation (mastitis) the device is preferably provided with four sampling elements arranged in parallel, i.e. one sampling element for each nipple of the investigated cattle.

For the purpose of further reducing the risk for contamination the device according to the invention is preferably provided with a hinged protective cover that can be pivoted forward in a direction towards the sampling location, said cover shielding the sampling parts against a contaminating environment when pushed out for sampling.

The invention also relates to a process for extracting samples from a liquid medium for the purpose of a later bacterial and/or ATP analysis.

It is commonly known that for example enzymes can be stabilized by drying onto for example paper. This stabilization is considered to take place due to the fact that when the water activity decreases below a critical level chemical wet reactions, such as inactivation or decomposition of biological molecules, cease. The process for stabilizing ATP and biological samples by drying onto for example paper have, however, not been tested. According to our experiments with drying of milk samples absorbed in paper it has been found that already drying results in a certain degree of stability. This stability can be improved by the addition of complex formers for two-valent metal ions, for example EDTA, and quaternary ammonium compounds, for example dodecyl trimethylammonium bromide, in order that an improved inhibition of ATP--destroying reactions shall be obtained in dried samples. Most ATP-converting enzymes are dependent on two-valent metal ions, such as Mg²⁺ and Ca²⁺, and also non-enzymatic ATP-decomposition is metal ion catalyzed which can explain the effect of EDTA. Quatenary ammonium compounds inactivate most enzymes and do so, as well as EDTA, without contribution to non-enzymatic decomposition of ATP. Enzymatic decomposition of ATP can be prevented with other agents, for example strong acids and bases, but such agents would result in non-enzymatic ATP-decomposition. In addition to quatenary ammonium compounds there are, however, also a number of enzyme-inactivating agents meeting the requirements that they shall not contribute to ATP-decomposition. To the skilled artisan it is a simple matter to find alternative enzyme-inactivating substances. Process of stabilizing ATP in biological samples by drying and preferably in the presence of complex formers for two-valent metal ions and/or substances inactivating enzymes without contributing to the non-enzymatic decomposition of ATP, constitutes one aspect of the invention.

It is also known that the drying of bacteria inhibits growth. In fact drying often results in the killing of most bacteria (however, spore-forming bacteria may survive strong drying by forming spores). Therefore, it was surprising when drying milk samples on paper only after a very long period of time gave a killing effect. Furthermore, the important advantage is gained in that growth of contaminating bacteria as well as bacteria present in the original sample will be prevented. The explanation why the killing in for example milk samples is such a slow process may be that complex biological samples contain many substances, for example proteins and fats, acting as protective colloids around the bacteria and preventing too strong drying. The use of an absorbing material, such as paper, as a matrix for absorbing the sample probably contributes to the stabilization, since cellulose fibres per se have the ability of binding water. Even if the sample is placed in absolutely dry air it will therefore take a very long time before all water has been removed from the sample and therefore the bacteria can survive. Samples containing too little of biological material that can protect against a too rapid drying can be supplemented with such material. It can be constituted by for example sterilized milk, since milk has been found to give a good stabilizing effect. In accordance with type of sample and analysis it should also be simple to find a synthetic stabilizer, for example some suitable mixture of protein, fat and/or carbohydrates. The process of stabilizing bacteria in biological samples by drying onto a solid carrier in the presence of endogeneous or added stabilizing substances constitutes another aspect of the invention.

The invention will in the following be further illustrated by exemplifying embodiments in association with the appended drawing, wherein:
Fig. 1 shows a plan view from above of a sampling device according to the invention;
Fig. 2 shows a section taken along line A-A in Fig. 1;
Fig. 3 shows an end view of the device of Fig. 1;
Fig. 4 shows a side view of a modified embodiment of the sampling device according to the invention; and
Fig. 5 shows a view from above of the device according to Fig. 4.

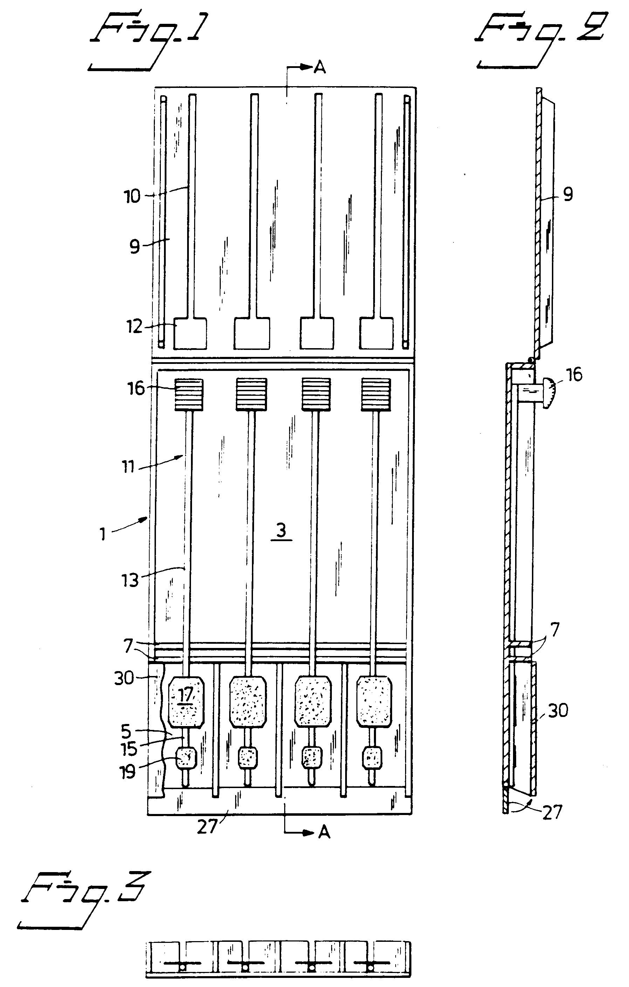

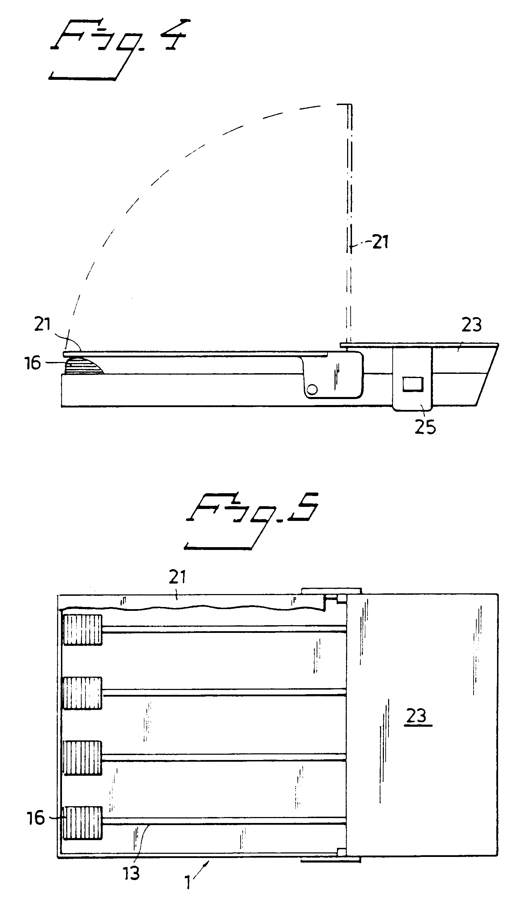

In Figs. 1 to 3 there is diagrammatically shown a device for sampling of freely flowing liquid media, for example milk from cows for test on mastitis. The device consists of a box or container generally designated 1 which is divided up into two spaces or compartments 3,5 separated by means of transverse partition walls 7. Compartment 3 is covered by a lid 9 provided with slots and recesses, 10 and 12, respectively.

The two chambers or compartments 3,5 accomodate sampling members generally designated 11, each comprising an operational part 13 placed in compartment 3 and a sampling part 15 placed in compartment 5. Each sampling member 15 for diagnosis on mastitis is provided with a sampling element 17 for bacterial analysis and a sampling element 19 for ATP-analysis. These sampling elements 17,19 can be constituted by an absorbing material, for example filter paper.

As is clear from Fig. 2 the device is furthermore provided with an outwardly pivotal end wall 27 which in a closed position during transportation protects sampling elements 17,19 against contamination through the slots 29 through which the sampling parts 15 can be extended out from the container or box 1. The device is furthermore provided with a lid 30 protecting sampling parts 15 in compartment 5. This lid 30 is removed in the view of the device shown in Fig. 1.

The embodiment according to Figs. 4 and 5 is provided with a hinged lid 21 which can be pivotally moved forward and which is intended to protect sampling part 15 in connection with the sampling procedure. Moreover, this embodiment is provided with a box 23 arranged above compartment 5, said box acting as a lid and being attachable to the device by means of anchoring lugs 25. This lid 23 in the form of a box contains a drying agent, for example silica gel, the presence of which means that samples taken can be transported under dry conditions to the laboratory. The drying agent can be easily removed through a lid provided on the box but not shown for replacement or for regeneration after a period of use. The bottom of the box facing the sampling compartment is perforated so that moisture from the samples can be easily taken up. Finally, this embodiment is provided with a support member 22 whereby the device when samples are taken can be manipulated with one hand.

The function of the device in practical use is briefly as follows.

Each sampling member 11 can after felling lid 27 be pushed forwardly out of compartment or chamber 5 by means of the grooved grip 16 extending up from the box. This will expose both sampling elements 17,19 so that sampling can take place by a jet of milk from one of the cow's nipples impinging on both sampling elements 17,19 so that milk will be absorbed therein. During this operation the protective lid 21 (Fig. 4) is moved to the position indicated with a dashed line so that the exposed sampling member 15 will not be substantially contaminated by surrounding material containing bacteria, for example hair or dirt from the cow etc. After terminated sampling operation the sampling part 15 used will be retracted into box 1, the procedure being repeated with the other sampling elements 11 while taking samples from the other nipples one by one.

The sampling device is after sampling transferred to a laboratory for analysis of the two sampling elements 17,19 which can be separated from the sampling part 15 of the sampling element in connection with the analysis.

The sampling device according to the invention has been tested with regard to samples of milk and of urine. The results of these analyses are given in appended Tables 1 and 2 relating to milk samples and urine samples, respectively, and these samples have been taken day 0. From the samples 200 µl have been transferred to filter paper and stored together with drying agent in the device according to the present invention at room temperature. The milk and urine samples have been tested day 0 with regard to amount and type of bacteria. The filter papers with the milk have been tested day 1 and 5 and those with urine day 2 and 5. The samples have been cultivated in a conventional manner.

As is clear from Table 1 the cultivation from milk day 0 shows the same bacteria as with storage on the filter paper for one and for five days. In certain samples the number of bacteria has decreased somewhat after five days. According to Table 2 the cultivation from urine day 1 shows the same type of bacteria as after storage for two days on the filter paper in eleven out of twelve samples. After storage for five days on the filter paper the same bacteria are present in nine out of twelve samples. The quantity of bacteria decreases when storing the urine sample more than for milk but the device according to the invention may still be practically used even for sampling of urine.

**TABLE 1**

| MILK SAMPLE | | | |
|---|---|---|---|
| Sample No | Cultivation Day 0 | Day 1 | Day 5 |
| 1 | 2SaR | 2SAR | 1SaR |
| 2 | 3SaR | 3SAR | 3SAR |
| 3 | 3EcR | 3EcR | 2EcR |
| 4 | 2,5SaR | 3SaR | 2SaR |
| 5 | 3SrdB | 3SrdB | 3SrdB |
| 6 | 3SrdB | 3SrdB | 2,5Srd(B) |
| 7 | 3SrdR | 3SrdR | 3SrdR |
| 8 | 0,5SaR | 0,5SaR | 0 |
| 9 | 1StafR | 1StafR | 1StafR |
| 10 | 2SröR | 2SröR | 1,5SröR |
| 11 | 1Sk-R | 1Sk-R | 1Sk-R |
| 12 | 3SrdB | 3SrdB | 2,5SrdB |
| 13 | 0,5Sk-R | 0,5Sk-R | 0 |

**TABLE 2**

| URINE SAMPLE | | | |
|---|---|---|---|
| Sample No | Cultivation Day 0 | Day 2 | Day 5 |
| 1 | 3Sk-R | 3SK-R | SK-R |
| 2 | 3Eöl/protB | 3EöB | 3Eöl/prot |
| 3 | 3EöB | 1EöB | 0 |
| 4 | 3Sk-R | 3SK-B | 3Sk-R |
| 5 | 3Sk-R | 3Sk-R | 2,5Sk-B |
| 6 | 3Eö,1protB | 2Eö, | 1Eö,1protB |
| | | 1protB | |
| 7 | 3EcR | 2EcR | 1EcR |
| 8 | 3EöB | 2,5EöB | 2,5EöB |
| 9 | 3Eö,2,5StafB | 3Eö, | 2Eö,2,5StafB |
| | | 3StafB | |
| 10 | 3EöR | 1EöB | 0 |
| 11 | 3StafR | 3StafR | 3StafR |
| 12 | 1EöB | 0 | 0 |
| 0-3 indicates quantity on an increasing scale R = pure culture B = mixed flora Sk = coagulationeg.staphylococcus prot.= proteus Eö = coliform bacteria Sa = Staph.aureus Ec = Escherichia coli Srd = Strept.dysgalactiae Staf = unspecific staphylococcus | | | |

## Claims

1. Device for sampling a liquid medium, comprising a container (1) which by a transverse partition (7) is divided into two compartments (3,5) containing at least one sampling element (11) comprising an operational part (13) placed in one compartment (3) and a sampling past (15) placed in the other compartment (5), the end wall of the container (1) in connection with said other compartment being provided with an opening (29) opposite to the sampling member (11) through which said member by means of the respective operational part (13) can be pushed out of the container (1) for exposing the sampling part (15) and for the uptake of sample thereon and then be retracted into the container (1) for protection during transportation, characterized in that said other compartment (5) is substantially closed against the environment and contains a drying agent.

2. Device according to claim 1, characterized by an openable box (23) containing drying agent which in mounted position seals said other compartment.

3. Device according to claim 1 or 2, characterized by an outwardly pivotal end wall (27) which during transportation with the sampling element (11) in a retracted position covers said openings (29).

4. Device according to claim 1, 2 or 3, characterized in that each sampling member (15) is provided with one or several detachable sampling elements (17,19) of an absorbant porous material.

5. Device according to claim 4, characterized in that each sampling member (15) is provided with two detachable sampling elements (17,19), one for for example bacterial analysis and another for for example ATP-analysis.

6. Device according to any preceding claim, characterized by a cover plate (9) which is provided with grooves (10) and recesses (12) placed opposite to the respective operational parts (13), said part being at its free end provided with a grip (16) extending up through the recess (12) for extension or retraction of the corresponding sampling member (15) in connection with sampling.

7. Device according to any preceding claim, characterized by several, for example four, sampling members (11) placed in parallel.

8. Device according to any preceding claim, characterized by a hinged protective cover (21) pivotal forwardly and intended to protect sampling members (15) against undesired contamination during sampling.

## Patentansprüche

1. Vorrichtung zur Probennahme eines flüssigen Mediums mit einem Behälter (1), der durch eine quer verlaufende Trennwand (7) in zwei Abteile (3, 5) geteilt ist, welche wenigstens ein Probennahmeelement (11) mit einem Arbeitsteil (13), das in einem Abteil (3) angeordnet ist, und einem Probennahmeteil (15), das in dem anderen Abteil (5) angeordnet ist, enthalten, wobei die Endwand des Behälters (1) in Verbindung mit dem anderen Abteil mit einer Öffnung (29) gegenüber dem Probennahmeteil (11) versehen ist, durch welche dieses Teil mit Hilfe des betreffenden Arbeitsteils (13) aus dem Behälter (1) zur Freigabe des Probennahmeteils (15) und zur Aufnahme einer Probe darauf gestoßen werden kann und dann in den Behälter (1) zum Schutz während eines Transportes zurückgezogen werden kann, **dadurch gekennzeichnet**, daß das andere Abteil (5) gegenüber der Umgebung im wesentlichen abgeschlossen ist und ein Trocknungsmittel enthält.

2. Vorrichtung nach Anspruch 1, **gekennzeichnet durch** einen öffenbaren Kasten (23), der Trocknungsmittel enthält, welches in befestigter Position das andere Abteil abdichtet.

3. Vorrichtung nach Anspruch 1 oder 2, **gekennzeichne**t **durch** eine nach außen schwenkbare Endwand (27), die während eines Transportes mit dem Probennahmeelement (11) in einer zurückgezogenen Position die Öffnungen (29) bedeckt.

4. Vorrichtung nach Anspruch 1, 2 oder 3, **dadurch gekennzeichnet**, daß jedes Probennahmeteil (15) mit einem oder mehreren lösbaren Probennahmeelementen (17, 19) eines porösen Absorbensmaterials versehen ist.

5. Vorrichtung nach Anspruch 4, **dadurch gekennzeichnet**, daß jedes Probennahmeteil (15) mit zwei lösbaren Probennahmeelementen (17, 19), einem beispielsweise für Bakterienanalyse und einem anderen beispielsweise für ATP-Analyse, versehen ist.

6. Vorrichtung nach einem der vorausgehenden Ansprüche, **gekennzeichnet durch** eine Deckplatte (9), die mit Nuten (10) und Vertiefungen (12) versehen ist, welche den betreffenden Arbeitsteilen (13) gegenüber angeordnet sind, wobei dieses Teil an seinem freien Ende mit einem Griff (16) versehen ist, der sich durch die Vertiefung (12) für ein Erstrecken oder Zurückziehen des entsprechenden Probennahmeteils (15) in Verbindung mit einer Probennahme erstreckt.

7. Vorrichtung nach einem der vorausgehenden Ansprüche, **gekennzeichnet durch** mehrere, beispielsweise vier Probennahmeteile (11) parallel zueinander.

8. Vorrichtung nach einem der vorausgehenden Ansprüche, gekennzeichnet durch einen nach vorn schwenkbaren und zum Schutz der Probennahmeteile (15) gegen unerwünschte Verunreinigung während der Probennahme bestimmten angelenkten Schutzdeckel (21).

## Revendications

1. Dispositif d'échantillonnage d'un milieu liquide, comprenant un réservoir (1) qui est divisé par une cloison transversale (7) en deux compartiments (3, 5) contenant au moins un élément d'échantillonnage (11) comprenant une partie opérationnelle (13) placée dans un premier compartiment (3) et une partie d'échantillonnage (15) placée dans l'autre compartiment (5), la paroi terminale du réservoir (1) en liaison avec ledit autre compartiment présentant, en regard de l'élément d'échantillonnage (11), une ouverture (29) à travers laquelle ledit élément peut être poussé hors du réservoir (1) par la partie opérationnelle respective (13) pour exposer la partie d'échantillonnage (15) et pour prélever l'échantillon de celle-ci puis retiré dans le réservoir (1) pour assurer sa protection au cours d'un transport, caractérisé en ce que ledit autre compartiment (5) est sensiblement fermé vis-à-vis de l'environnement et contient un agent dessicant.

2. Dispositif selon la revendication 1, caractérisé par une boîte ouvrable (23) contenant l'agent dessicant, qui, en position de montage, scelle ledit autre compartiment.

3. Dispositif selon la revendication 1 ou 2, caractérisé par une paroi terminale (27) pivotante vers l'extérieur qui recouvre lesdites ouvertures (29) lorsque l'élément d'échantillonnage (11) est en position de retrait en cours de transport.

4. Dispositif selon la revendication 1, 2 ou 3, caractérisé en ce que chaque élément d'échantillonnage (15) est muni d'un ou plusieurs éléments d'échantillonnage détachables (17, 19) constitués d'une matière poreuse absorbante.

5. Dispositif selon la revendication 4, caractérisé en ce que chaque élément d'échantillonnage (15) est muni de deux éléments d'échantillonnage détachables (17, 19), un pour, par exemple, une analyse bactériologique et un autre, par exemple, pour une analyse ATP.

6. Dispositif selon l'une quelconque des revendications précédentes, caractérisé par une plaque de couverture (9) qui comporte des rainures (10) et des évidements (12) placés en regard des parties opérationnelles respectives (13), ladite partie étant munie, à son extrémité libre, d'un élément de préhension (16) s'étendant à travers l'évidement (12) pour pouvoir étendre ou retirer l'élément d'échantillonnage correspondant (15) en liaison avec l'échantillon.

7. Dispositif selon l'une quelconque des revendications précédentes, caractérisé par plusieurs éléments d'échantillonnage (11), par exemple, quatre, placés en parallèle.

8. Dispositif selon l'une quelconque des revendications précédentes, caractérisé par un couvercle protecteur à charnière (21) susceptible de pivoter vers l'avant et destiné à protéger les éléments d'échantillonnage (15) contre toute contamination involontaire au cours de l'échantillonnage.
